(19) Europäisches Patentamt
European Patent Office
Office européen des brevets

(11) EP 1 266 970 B1

(12) **EUROPEAN PATENT SPECIFICATION**

(45) Date of publication and mention of the grant of the patent:
**24.09.2008 Bulletin 2008/39**

(51) Int Cl.:
***C12Q 1/68*** *(2006.01)*

(21) Application number: **02254040.5**

(22) Date of filing: **11.06.2002**

(54) **Detecting nucleic acid deletion sequences**

Detektion von Nukleinsäure-Deletionssequenzen

Détécter des séquences d'acides nucléiques délétés

(84) Designated Contracting States:
**AT BE CH CY DE DK ES FI FR GB GR IE IT LI LU MC NL PT SE TR**

(30) Priority: **11.06.2001 US 877748**

(43) Date of publication of application:
**18.12.2002 Bulletin 2002/51**

(73) Proprietor: **ORTHO-CLINICAL DIAGNOSTICS, INC.**
**Rochester, NY 14626-5101 (US)**

(72) Inventor: **Sutherland, John W.**
**Rochester, NY 14620 (US)**

(74) Representative: **Mercer, Christopher Paul et al**
**Carpmaels & Ransford**
**43, Bloomsbury Square**
**London WC1A 2RA (GB)**

(56) References cited:
**WO-A-96/32500**
**US-A- 5 750 345**
**US-A- 5 776 682**

- **FUKAGAWA N K ET AL: "Aging and high concentrations of glucose potentiate injury to mitochondrial DNA" FREE RADICAL BIOLOGY AND MEDICINE 1999 UNITED STATES, vol. 27, no. 11-12, 1999, pages 1437-1443, XP002258115 ISSN: 0891-5849**
- **MARIN-GARCIA J ET AL: "Specific mitochondrial DNA deletions in idiopathic dilated cardiomyopathy" CARDIOVASCULAR RESEARCH 1996 NETHERLANDS, vol. 31, no. 2, 1996, pages 306-313, XP002258116 ISSN: 0008-6363**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> Ortho-Clinical Diagnostics, Inc.

<120> Detecting Nucleic Acid Deletion Sequences

<130> P030663EP

<140> EP 02254040.5
<141> 2002-06-11

<150> US 09/877,748
<151> 2001-06-11

<160> 26

<170> PatentIn version 3.1

<210> 1
<211> 107
<212> DNA
<213> Artificial Sequence

<400> 1
cacagacatc ataacaaaaa atttccacca aaccccccct cccccgcttc tggccacagc 60
acttaaacac atctctgcca aaccccaaaa acaaagaacc ctaacac 107

<210> 2
<211> 24
<212> DNA

<213> Artificial Sequence

<400> 2
cacagacatc ataacaaaaa attt 24

<210> 3
<211> 25
<212> DNA
<213> Artificial Sequence

<400> 3
gtgttagggt tctttgtttt tgggg 25

<210> 4
<211> 34
<212> DNA
<213> Artificial Sequence

<400> 4
gcgagctctg gccacagcac ttaaacccgc tcgc 34

<210> 5

<211> 330
<212> DNA
<213> Homo sapiens

<400> 5

```
agaaccaaca cctctttaca gtgaaatgcc ccaactaaat actaccgtat ggcccaccat     60
aattaccccc atactcctta cactattcct catcacccaa ctaaaaatat taaacacaaa    120
ctaccaccta cctccctcac caaagcccat aaaaataaaa aattataaca aaccctgaga    180
accaaaatga acgaaaatct gttcgcttca ttcattgccc ccacaatcct aggcctaccc    240
gccgcagtac tgatcattct atttcccct ctattgatcc ccacctccaa atatctcatc     300
aacaaccgac taatcaccac ccaacaatga                                     330
```

<210> 6
<211> 27
<212> DNA
<213> Artificial Sequence

<400> 6
accaacacct ctttacagtg aaatgcc 27

<210> 7
<211> 21
<212> DNA
<213> Artificial Sequence

<400> 7
tcattgttgg gtggtgatta g 21

<210> 8

<211> 35
<212> DNA
<213> Artificial Sequence

<400> 8
ccgtcgcctc cctcaccaaa gcccataaac gacgg 35

<210> 9
<211> 28
<212> DNA
<213> Artificial Sequence

<400> 9
tgtatgatat gtttgcggtt tcgatgat 28

<210> 10
<211> 20
<212> DNA

<213> Artificial Sequence

<400> 10
gccccaacta aatactaccg 20

<210> 11
<211> 26
<212> DNA
<213> Artificial Sequence

<400> 11
gatgtggtct ttggagtaga aacctg 26

<210> 12
<211> 46
<212> DNA
<213> Artificial Sequence

<400> 12
ccgctcgaaa ggtattcctg ctaatgctag gctgccaatc gagcgg 46

<210> 13
<211> 181
<212> DNA
<213> Homo sapiens

<400> 13

```
ggggaagcag atttgggtac cacccaagta ttgactcacc catcaacaac cgctatgtat      60
ttcgtacatt actgccagcc accatgaata ttgtacggta ccataaatac ttgaccacct     120
gtagtacata aaaacccaat ccacatcaaa accccctccc catgcttaca agcaagtaca     180
g                                                                     181
```

<210> 14
<211> 22

<212> DNA
<213> Artificial Sequence

<400> 14
ggggaagcag atttgggtac ca 22

<210> 15
<211> 20
<212> DNA
<213> Artificial Sequence

<400> 15
ctgtacttgc ttgtaagcat 20

<210> 16
<211> 36
<212> DNA
<213> Artificial Sequence

<400> 16
gcgtcggact cacccatcaa caaccgctat cgacgc 36

<210> 17
<211> 198
<212> DNA
<213> Homo sapiens

<400> 17

```
gccgcagtac tgatcattct atttcccct ctattgatcc ccacctccaa atatctcatc     60

aacaaccggc tatgtatttc gtacattact gccagccacc atgaatattg tacggtacca    120

taaatacttg accacctgta gtacataaaa acccaatcca catcaaaacc ccctccccat    180

gcttacaagc aagtacag                                                  198
```

<210> 18
<211> 36
<212> DNA
<213> Artificial Sequence

<400> 18
gcgtcgctgc cagccaccat gaatattgta cgacgc 36

<210> 19
<211> 33
<212> DNA
<213> Artificial Sequence

<400> 19
gccgcagtac tgatcattct atttccccct cta 33

<210> 20
<211> 47
<212> DNA
<213> Artificial Sequence

<400> 20
ccgctcggcc gcagtactga tcattctatt tccccctcta cgagcgg 47

<210> 21
<211> 30
<212> DNA
<213> Artificial Sequence

<400> 21
tactcaaaac catacctctc acttcaacct 30

<210> 22
<211> 30
<212> DNA
<213> Artificial Sequence

<400> 22
gaacgaaaat ctgttcgctt cattcattgc 30

<210> 23
<211> 39
<212> DNA
<213> Artificial Sequence

<400> 23
gcgtcgcatc acccaactaa aaatattaaa caccgacgc 39

<210> 24
<211> 22
<212> DNA
<213> Artificial Sequence

<400> 24
ctgctaatgc taggctgcca at 22

<210> 25
<211> 53
<212> DNA
<213> Artificial Sequence

<400> 25
cgccgcctca tcacccaact aaaaatatta aacacaaact accaccggcg gcgv 53

<210> 26
<211> 43
<212> DNA
<213> Artificial Sequence

<400> 26
cctgcggctg ccagccacca tgaatattgt acggtaccgc agg 43

## Description

## BACKGROUND

This invention relates to determining the presence and/or abundance of mutant (Mutant) nucleic acids in a sample of interest.

Mitochondria are cytoplasmic organelles distributed in animal cells whose principal function is to generate energy-rich ATP molecules necessary for driving cellular biochemical processes. Mitochondria contain their own DNA that is separate and distinct from chromosomal DNA. Mitochondrial DNA ("mt-DNA") encodes exclusively for a number of critical protein subunits of the electron transport chain and the structural rRNAs and tRNAs necessary for the expression of these proteins. Unlike chromosomal DNA, each cell can contain 100,000 copies or more of mt-DNA. Cells can harbor mixtures of wild-type and mutant mt-DNA (heteroplasmy). Mitochondrial genes are dynamic and the mt-DNA genotype can drift towards increased mt-DNA mutational burden in heteroplasmic cellular populations. The metabolic phenotype can deteriorate with time under these conditions, and can result in disease manifestation once the mutational burden exceeds a critical threshold in affected tissue, leading to bioenergetic failure and eventually cell death.

Mitochondrial disorders are particularly problematic in organs such as the heart and the brain, the two organs with the highest metabolic requirements for energy and the highest abundance of mitochondria (mt). ATP synthesis requires oxygen ("oxidative phosphorylation"); hence, acute hypoxia can be especially damaging to these tissues. Periods of hypoxia followed by resupply of oxygen can cause the release of oxygen-derived free radicals; these radicals can damage DNA (perfusion/reperfusion injury). Such radicals are generated in abundance by the mitochondrial cytochromes, located in close proximity to the mitochondrial genome. Moreover, since mitochondria are derived from primitive bacteria they lack the more advanced repair mechanisms found in the mammalian nucleus. For both reasons mitochondrial DNA undergoes higher mutation rates than the nuclear genome.

The activities of enzymes associated with oxidative phosphorylation (OXPHOS) have been shown to decline with age in human and primate muscle, liver, and brain. This is paralleled by an age-related increase in heart and skeletal muscle fiber focal cytochrome oxidase (COX) deficiency, with the COX-negative regions containing clonal expansions of individual mt-DNA rearrangements. It is also correlated with the accumulation of a variety of somatic mt-DNA mutations, including various deletions and base substitutions. The extent of mt-DNA damage that accumulates in various tissue is correlated with those tissues most prone to age-related dysfunction. Thus the basal ganglia accumulates the highest levels of mt-DNA damage, followed by the various cortical regions. Yet the cerebellum remains relatively free of mt-DNA damage throughout life. This suggests that the accumulation of somatic mt-DNA mutations may be an important factor in the age-related decline of somatic tissues.

As somatic mutations accumulate they could exacerbate inherited OXPHOS defects until the combined defect is sufficient to result in energetic failure of the tissues. Three late-onset progressive diseases associated with an increased frequency of somatic mt DNA mutations are: i) late-onset (>69 years) mitochondrial myopathy involving insidious proximal muscle (limb-girdle) weakness with fatigability; ii) inclusion body myositis involving late-onset chronic inflammatory muscle disease resulting in muscle weakness; and, iii) polymyalgia rheumatica associated with inflammatory stiffness and pain in the scapular and pelvic girdles. OXPHOS defects have also been reported in Huntington's Disease (HD), dystonia, and Alzheimer's Disease (AD). Somatic mt-DNA mutations have been reported to be elevated in sun-exposed skin, certain types of cardiomyopathy, livers of alcoholics, ovaries of post-menopausal women, and reduced mobility sperm.

Many mutations to mt-DNA are well known and characterized. Recently, large (kb long) mt-DNA genomic deletions have been found in cases involving certain ischemic heart diseases, cardiomyopathies, and myoclonic epilepsy. So-called "long" PCR has been the method of choice for unveiling such mutations. In this technique, the entire mitochondrial genome of damaged or affected tissue is amplified. This full-length mt-DNA is then sequenced and compared with the known sequence of intact human mt-genome to identify the missing nucleotide sequences.

While useful as a research tool, long PCR is not an efficient clinical method in this context. Moreover, a clinically useful method must minimize amplification or signaling attributable to nuclear pseudogenes. These are nucleic acid sequences similar to mt-DNA sequences that have been incorporated into the DNA of the nucleus. Additionally, long-PCR is tedious requiring highly specialized laboratory skills. If not carefully practiced, long-PCR can readily produce false negative results.

Other methods for characterizing particular aspects of DNA molecules are known as well. For example, U.S. Patent 5,436,142 to Wigler proposes a method for producing probes for distinguishing different DNA molecules of similar origin. In this method, DNA molecules from two different but related sources are cleaved with restriction enzymes. The restriction fragments are amplified. Both sets of amplicons are then mixed together, melted, annealed and amplified. This is done with an excess of the amplicons originating from the DNA missing a particular sequence (the "target"). The product of this process is an enriched DNA molecule containing the target. These molecules can then be used to prepare probes for polymorphism sequences that are complementary to the target.

U.S. Patent 5,919,623 to Taylor proposes the intentional construction of a heteroduplex DNA using restriction fragments of different DNAs, one of which is suspected of having a sequence of interest. The heteroduplex so obtained contains a mismatch. This mismatch can then be identified using a mismatch repair protein that binds to the mismatch site. Similarly, U.S. Patent 6,110,684 to Kember employs a resolvase to detect mismatches. U.S. Patent 5,391,480 to Davis also identifies the presence of a sequence of interest through the creation of a mismatch. In this case an exonuclease is used to cleave a label at the site of the mismatch if a mismatch is found. U.S. Patent 5,958,692 to Cotton is yet another patent directed to the detection of heteroduplex mismatches. The method employs a resolvase system that cleaves cruciform DNA molecules. Experimental results in the patent indicated that the method was unable to detect three out of the four mutations that were deletion sequence mutations. It does not correlate amplification with the lack of a sequence. U.S. Patent 5,876,941 to Landegren is similar to the Cotton patent in many respects.

U.S. Patent 6,001,567 to Brow proposes the use of a modified DNA polymerase to identify target DNA sequences. The polymerase is modified so that it retains 5' exonuclease activity but no longer has any synthetic ability. Nucleic acid segments are crafted so that they contain sequences that will flank the target sequence. One such segment contains a 5' arm that is subject to attack and cleavage by the modified polymerase when a specific portion of the remainder of the DNA molecule to which it is attached binds to the target sequence. Cleavage of the 5' arm initiates a signaling process indicating the presence of the target sequence. In this case, the agent responsible for locating the target sequence is the nucleic acid that is subject to cleavage. The modified DNA polymerase has no direct role in locating the target sequence.

U.S. Patent 6,017,701 to Sorge proposes a method for preferentially amplifying nucleic acids having certain discrete sequences. In this method, nucleic acids are modified with adapters that also serve as primers for amplification. The adapters that bind to nucleic acids that do not have the sequences that are to be enriched also have sequences that are subject to attack by restriction enzymes. Thus, nucleic acids that lack the sequences of interest cannot be amplified while those that have such sequences are amplified. The patent does not propose the amplification of nucleic acids that are mutant such that they lack certain sequences. Further, distinguishing nucleic acids based on whether or not a given sequence is present does not occur until priming has already occurred.

WO 9632500 to Todd proposes a method of detecting a genetic polymorphism in an individual. This is done by PCR amplifying the sample using primers selected so that they introduce into the wild type amplicon a site cleaved by a restriction enzyme, so that this wild type DNA is not amplified. This Restriction Endonuclease Mediated PCR permits the selective enrichment of the mutant DNA present in a large excess of wild type DNA.

Most of the techniques previously described apply primarily to the detection of point or short mutations. Detecting deletions is particularly arduous in that they are sequences that are missing in the mutant molecule. Essentially, one is trying to detect a small fraction of DNA that is missing a sequence in the presence of a large excess of wild type DNA that contains that sequence. Yet diagnostics are necessary where a low level of the deletions may be clinically relevant. Thus, commercially available diagnostics for identifying such mutations should be sensitive, specific, robust and quantitative. Although sequencing has been used by others to establish the clinical relevance of these deletions, sequencing *per se* is too complex for commercial diagnostics.

Methods for identifying deletions in mitochondrial DNA are described in Marin-Garcia, J. et al. (Cardovascular Research 31 (1996) 306-313) and Fukugawa, N.M. et al. (Free Radical Biology and Medicine, vol. 27, Nos. 11/12 (1999) 1437-1443). The methods involve detecting mutant nucleic acid by contacting a nucleic acid sample with primers complementary to priming sites outside of a deleted region of DNA and amplifying the product under short PCR conditions.

## SUMMARY OF INVENTION

The invention is a method for determining the presence of deletion mutations in nucleic acids. In this method, a sample having nucleic acid present is contacted with mutant PCR primers under short PCR conditions. The nucleic acid that has been contacted with this material is then amplified and identified. Nucleic acids having long sequences between the sequences that hybridize to the mutant PCR primers (such as wild type DNA) are not amplified. Thus, the presence of amplicons indicates the presence of nucleic acid sequences having sequences deleted from the wildtype nucleic acid.

A sample having nucleic acid present is contacted with a cleavage reagent. The nucleic acid that has been contacted with this material is then amplified and identified. Nucleic acids that do not have the sequence that the material attacks are not cleaved and are therefore amplified under the correct amplifying conditions. Wild type nucleic acids having the sequence that the material attacks, are cleaved, and are not amplified by the mutant primers that flank the cleavage point.

The cleavage reagents include, for example, a restriction enzyme specific for the deletion sequence, a DNAzyme, Ribozyme or other material with requisite specificity.

In one embodiment of the invention the nucleic acid subjected to this method is mitochondrial DNA (mt-DNA).

The method includes the step of preparing multiple aliquots of sample. One aliquot is amplified to detect the presence of nucleic acid having deletions and the other is amplified to detect the presence of wild type nucleic acid. The amplicons from this process are useful as a positive control. In a further embodiment of the invention, the method is quantitated by comparing the quantity of the mutant nucleic acid with the quantity of the nonmutant nucleic acid.

Molecules useful as primers and probes for conducting the methods described above are described.

In yet another embodiment of the invention, a kit is provided that includes amplification reagents and cleavage reagents.

The inventions described in this specification are useful in commercial clinical diagnostic applications.

## BRIEF DESCRIPTION OF THE DRAWINGS

Fig. 1 shows a schematic representation of one method of practicing the invention.

Fig. 2 shows a schematic representation of a method of practicing the invention different from that presented in Fig. 1.

## DETAINED DESCRIPTION

### Definitions:

"Wildtype (wt) nucleic acid sequence" is the nucleic acid sequence that is considered the standard for the organism with respect to genotype and phenotype. It is the sequence that is most prevalent for a given gene or coding segment for the organism as it is seen in the wild. In the context of this specification, the term refers to a segment of nucleic acid that is not mutated with deletions of long sequences of bases.

"Mutant nucleic acid sequence" is a nucleic acid sequence that deviates from the wildtype sequence. Deletion sequences are thus mutants according to this view since they deviate from the wildtype sequence.

"Nucleic acid deletion sequence" is a nucleotide sequence or segment that is present in a wildtype nucleic acid sequence for an organism and whose absence in a nucleic acid segment of interest for a given individual or group constitutes a mutation. That is, the absence of the sequence makes the nucleic acid segment a mutant nucleic acid sequence. Generally, such mutations can be manifested within an individual in a clinically significant way such as with an illness or a predisposition to illness.

"Cleavage reagents" are materials that when contacted with nucleic acids cut them at a particular site or sites. Restriction enzymes are the most preferred cleavage reagents but DNAzymes and other reagents that degrade or attack the nucleic acid at a particular site can also be used (provided they do not also degrade the mutant DNA or otherwise render it incapable of amplification). In the kits and methods of this invention, the cleavage reagents shear, degrade, and/or attack a nucleic acid having a known nucleic acid deletion sequence. For example, an assay for the detection of a Mutant mt-DNA missing a certain 5kb deletion sequence would include cleavage reagents that cleave wt mt-DNA at one or more points within the deletion sequence or as a result of having the deletion sequence so as to prevent amplification of the wild type sequence when mutant primers are used in the amplification.

"Cleave" and "cleavage" as the terms are used in relation to the use of cleavage reagents refers to a process by which a nucleic acid is rendered incapable of amplification when the cleavage point is flanked by a forward and a reverse priming site, as is the case here for the primers used for detecting deletion sequences.

"Short PCR" or "sPCR" means amplification via the polymerase chain reaction (PCR) wherein the sequences that are amplified are less than about 1kb, preferably 500 bases or less, and most preferably 300 bases or shorter. Short PCR can be ensured by the practice of PCR under certain conditions described in detail below.

"Long PCR" means amplification via the polymerase chain reaction (PCR) wherein the sequences that are amplified are greater than about 5kb. Long PCR involves the practice of PCR under certain conditions described in detail below.

"Primer", as the term is used throughout this specification has its ordinary meaning within the context of PCR technology.

"mutant PCR Primers" means primers made to hybridize to primer sites on complementary nucleic acid sequences that are no less than 1kb apart for the wild type DNA.

"wild type PCR Primers" means primers made to hybridize to primer sites on complementary nucleic acid sequences that are no greater than 1kb apart for the wild type DNA.

### Assay Targets

The targets of the assays of this invention are nucleic acids suspected of having mutations comprising deletions of no less than 4 kb. Preferably the targets are suspected of having deletions of about 4kb to about 10kb. Most preferably, the targets are suspected of having deletions of about 5kb to about 7 kb.

While the nucleic acids that are assayed according to this invention may be of any origin, mt-DNA is particularly amenable to the methods of this invention given its high mutation rate, the number and type of known prominent deletions, and the clinical significance of the quantitation of mt-DNA. Moreover, the length of the mt-DNA genome and the nature of the distribution of deletions in mutant mt-DNA make it an excellent analyte for the assays of this invention. The mt-DNA of the preferred embodiments can come from any source of human mt-DNA including whole blood and tissues (such as placental sample).

mt-DNA suspected of having the following deletions are preferred analytes (c.f. www.gen.emory.edu/mitomap; the Emory University website on mt-DNA):

| Deletion Junction (nt:nt) | Deletion Size(bp) | Genes Deleted* |
|---|---|---|
| 470:5152 | -4681 | MTTFH- MTND2 |
| 502:5443 | -4939 | MTTFH- MTND2 |
| 547:4443 | -3895 | MTHSP1- MTTM |
| 1836:5447 | -3610 | MTRNR2- MTND2 |
| 3173:14161 | -10987 | MTRNR2- MTND6 |
| 4398:14822 | -10422 | MTTQ- MTCYB |
| 5786:13923 | -8136 | MTTC- MTND5 |
| 5793:12767 | -6973 | MTTC- MTND5 |
| 5835:12661 | -6825 | MTND5- MTTY |
| 6023:14424 | -8400 | MTCO1-MTND6 |
| 6074:9179 | -3104 | MTCO1- MTATP6 |
| 6075:13799 | -7723 | MTCO1- MTND5 |
| 6226:13456 | -7279 | MTCO1- MTND5 |
| 6238:14103 | -7864 | MTCO1- MTND5 |
| 6325:13989 | -7663 | MTCO1- MTND5 |
| 6329:13994 | -7664 | MTCO1- MTND5 |
| 6330:13994 | -7663 | MTCO1- MTND5 |
| 6380:14096 | -7715 | MTCO1- MTND5 |
| 6465:14135 | -7669 | MTCO1- MTND5 |
| 7193:14596 | -7402 | MTCO1- MTND6 |
| 7438:13476 | -6037 | MTCO1- MTND5 |
| 7449:15 926 | -8476 | MTFS1- MTTT |
| 7491:11004 | -3512 | MTTS1- MTND4 |
| 7493:12762 | -5268 | MTTS1- MTND5 |
| 7501:14428 | -6926 | MTND6- MTTS1 |
| 7635:15440 | -7804 | MTCO2- MTCYB |
| 7669:15437 | -7767 | MTCO2- MTCYB |
| 7697:12364 | -4666 | MTCO2- MTCYB |
| 7777:13794 | -6016 | MTCO2- MTND5 |
| 7808:14799 | -6990 | MTCO2- MTCYB |
| 7815:15381 | -7565 | MTCO2- MTCYB |
| 7829:14135 | -6305 | MTCO2- MTND5 |
| 7841:13905 | -6063 | MTCO2- MTND5 |
| 7841:13905 | -6063 | MTCO2- MTND5 |
| 7845:9748 | -1902 | MTCO2- MTCO3 |
| 7974:15496 | -7521 | MTCO2- MTCYB |
| 8032:16075 | -8042 | MTCO2- MTATT |
| 8210:15339 | -7128 | MTCO2- MTCYB |
| 8213:13991 | -5777 | MTCO2- MTND5 |
| 8278:13770 | -5491 | MTTK- MTND5 |
| 8304:15055 | -6750 | MTTK- MTCYB |
| 8412:15664 | -7251 | MTATP8- MTTT |

(continued)

| Deletion Junction (nt:nt) | Deletion Size(bp) | Genes Deleted* |
|---|---|---|
| 8426:12894 | -4467 | MTND5- MTATP8 |
| 8468:13446 | -4977 | MTND5- MTATP8 |
| 8469:13447 | -4977 | MTND5- MTATP8 |
| 8482:13460 | -4977 | MTATP8- MTND5 |
| 8517:15421 | -6903 | MTATP8- MTCYB |
| 8563:13758 | -5196 | MTATP6- MTND5 |
| 8563:14596 | -6032 | MTATP6- MTND6 |
| 8570:13236 | -4665 | MTATP8- MTCYB |
| 8573:15727 | -7153 | MTATP8- MTCYB |
| 8580:15731 | -7150 | MTATP6- MTCYB |
| 8582:15957 | -7374 | MTATP6-MTTP |
| 8623:15662 | -7038 | MTATP6- MTCYB |
| 8624:13886 | -5261 | MTATP6- MTND5 |
| 8631:13513 | -4881 | MTATP6- MTND5 |
| 8637:16084 | -7446 | MTATP6-MTTP |
| 8648:16085 | -7436 | MTATP6- MTTP |
| 8707:13723 | -5015 | MTATP6-MTND5 |
| 8823:15855 | -7031 | MTATP6- MTCYB |
| 8828:14896 | -6067 | MTATP6- MTCYB |
| 8992:16072 | -7079 | MTATP6- MTTP |
| 9144:13816 | -4671 | MTATP6- MTND5 |
| 9180:14281 | -5100 | MTATP6- MTND6 |
| 9191:12909 | -3717 | MTATP6- MTND5 |
| 9238:15576 | -6377 | MTCO3- MTCYB |
| 9320:14273 | -4952 | MTCO3 MTND6 |
| 9357:13865 | -4507 | MTCO3- MTND5 |
| 9515:13055 | -3539 | MTCO3- MTND5 |
| 9574:12972 | -3397 | MTCO3- MTND5 |
| 9995:15897 | -5901 | MTTG- MTTT |
| 10050:15076 | -5025 | MTTG- MTCYB |
| 10058:14593 | -4534 | MTND6- MTND3 |
| 10154:15945 | -5790 | MTND3- MTTT |
| 10169:14435 | -4265 | MTND3- MTND6 |
| 10190:13753 | -3562 | MTND3- MTND5 |
| 10367:12829 | -2461 | MTND3- MTND5 |
| 10370:15570 | -5199 | MTND3- MTCYB |
| 10587:15913 | -5325 | MTND4L- MTTT |
| 10598:13206 | -2607 | MTND4L- MTND5 |
| 10665:14856 | -4190 | MTND4L- MTCYB |
| 10676:14868 | -4191 | MTND4L- MTCYB |
| 10744:14124 | -3379 | MTND4L- MTND5 |
| 10941:15362 | -4420 | MTND4- MTCYB |
| 10952:15837 | -4884 | MTND4- MTCYB |
| 10961:15846 | -4884 | MTND4- MTCYB |
| 11232:13980 | -2747 | MTND4- MTND5 |
| 11368:15786 | -4417 | MTND4- MTCYB |
| 12102:14412 | -2309 | MTND4- MTND6 |
| 12103:14414 | -2310 | MTND4- MTND6 |
| 12113:14422 | -2308 | MTND4- MTND6 |
| 12203:15355 | -3151 | MTTH- MTCYB |

Note: terminology adopted in Table 1 is the same as that adopted in MITOMAP (see below): MITOMAP: Mitochondrial DNA Function Locations

| Map Locus | Map Position(np) | Shorthand | Description |
|---|---|---|---|
| MTOHR | 110-441 | OH | H-strand origin |
| MTCSB1 | 213-235 | CSB1 | Conserved Sequence Block I |
| MTTFX | 233-260 | | mtTF1 binding site |
| MTTFY | 276-303 | | mtTF1 binding site |
| MTCSB2 | 299-315 | CSB2 | Conserved Sequence Block II |
| MTHPR | 317-321 | | |

replication primer

MTCSB3

346-363

CSB3

Conserved Sequence Block III

MTMT4H

371-379

mt4 H-strand control element

MTMT3H

384-391

mt3 H-strand control element

MTLSP

392-445

PL

L-strand promoter

MTTFL

418-445

mtTF1 binding site

MTTFH

523-550

mtTF1 binding site

MTHSP1

545-567

PH1

Major H-strand promoter

MTTF

577-647

F

tRNA Phenylalanine

MTHSP2

645-645

PH2

Minor H-strand promoter

MTRNR

648-1601

12S

12S rRNA

MTTV

1602-1670

V

tRNA Valine

MTRNR2

1671-3229

165

168 rRNA

MTRNR3

3206-3229

55-like sequence

MTTER

3229-3256

Transcription terminator

MTTL1

3230-3304

L(UUA/G)

tRNA Leucine 1

MTNC1

3305-3306

non-coding nucleotides between MTTL1 and MTND1

MTND1

3307-4262

ND1

NADH dehydrogenase 1

MTTI

4263-4331

I

tRNA Isoleucine

MTTQ

4329-4400

Q

tRNA Glutamine

MTNC2

4401-4401

non-coding nucleotide between MTTQ and MTTM

MTTM

4402-4469

M

tRNA Methionine

MTND2

4470-5511

ND2

NADH dehydrogenase 2

MTTW

5512-5576

W

tRNA Tryptophan

MTNC3

5577-5586

non-coding nucleotides between MTTW and MTTA

MTTA

5587-5655

A

tRNA Alanine

MTNC4

5656-5656

non-coding nucleotide between MTTA and MTTN

MTTN

5657-5729

N

tRNA Asparagine

MTOLR

5721-5798

OL

L-strand origin

MTTC

5761-5826

C

tRNA Cysteine

MTTY

5826-5891

Y

tRNA Tyrosine

MTNC5

5892-5903

non-coding nucleotides between MTTY and MTCO1

MTCO1

5904-7445

COI

Cytochrome c oxidase I

MTTS1

7445-7516

S(UCN)

tRNA Serine 1

MTNC6

7517-7517

non-coding nucleotide between MTTS1 and MTTD

MTTD

7518-7585

D

tRNA Aspartic acid

MTCO2

7586-8269

COII

Cytochrome c oxidase II

MTNC7

8270-8294

non-coding nucleotides between MTCO2 and MTTK

MTTK

8295-8364

K

tRNA Lysine

MTNC8

8365-8365

non-coding nucleotide between MTTK and MTATP8

MTATP8

8366-8572

ATPase8

ATP synthase 8

MTATP6

8527-9207

ATPase6

ATP synthase 6

MTCO3

9207-9990

COIII

Cytochrome c oxidase III

MTTG

9991-10058

G

tRNA Glycine

MTND3

10059-10404

ND3

NADH dehydrogenase 3

MTTR

10405-10469

R

tRNA Arginine

MTND4L

10470-10766

ND4L

NADH dehydrogenase 4L

MTND4

10760-12137

ND4

NADH dehydrogenase 4

MTTH

12138-12206

H

tRNA Histidine

MTTS2

12207-12265

S(AGY)

tRNA Serine2

MTTL2

12266-12336

L(CUN)

tRNA Leucine2

MTND5

12337-14148

ND5

NADH dehydrogenase 5

MTND6

14149-14673

ND6

NADH dehydrogenase 6

MTTE

14674-14742

E

tRNA Glutamic acid

MTNC9

14743-14746

non-coding nucleotides between MTTE and MTCYB

MTCYB

14747-15887

Cytb

Cytochrome b

MTTT

15888-15953

T

tRNA Threonine

MTATT

15925-499

membrane attachment site

MTNC10

15954-15954

non-coding nucleotide between MTTT and MTTP

MTTP

15955-16023

P

tRNA Proline

MTDLOOP

16028-577

D-Loop

Displacement Loop (see note below)

MT7SDNA

16106-191

7S

7S DNA

MTTAS

16157-16172

TAS

termination sequence

MTMT5

16194-16208

mt5

control element

MTMT3L

16499-16506

mt3

L-strand control element

"D-Loop" in this database refers to the non-coding region between Proline and Phenylalanine (np16024-576)

Locus names are the official designations delineated by the given nucleotide numbers. The map positions correspond to the nucleotide pair(np) numbers determined from the DNA sequence. The map symbols are used to indicate the position of the locus on the map.

Notes further define each locus: TAS=termination associated sequence, CSB=conserved sequence block, mtTF1=mitochondrial transcription factor, Y=either pyrimidine, N=any base. H-strand replication origin positions have been identified at np 110, 147, 169, 191, 219, 310, 441. L-strand promoter positions have been identified at np 407, 392-435. H-strand promoter positions have been identified at np 559 1, 561. L-strand replication origin positions have been identified at np 5721-5781, 5761, 5799.

## Sample Preparation

Well known methods for obtaining nucleic acids from a variety of sources (preferably human mt-DNA) can be used to obtain and isolate nucleic acids for use in the methods of this invention. For example, the DNA from a blood sample may be obtained by cell lysis following alkali treatment. Total nucleic acid can be obtained by lysis of white blood cells resuspended in water by incubation of cells in a boiling water bath for about 10 minutes. After cooling, cellular debris is removed, such as by centrifugation at about 14,000 g for about two minutes. The clear supernatant, which contains the DNA, may be stored frozen, e.g., at about -80.degree. C.

Where the target is mt-DNA, the conventional proteinase K/phenol-chloroform methods of isolating DNA are not preferred nor are other conventional methods of DNA isolation. Instead, the boiling method described above is preferred. This is described more fully in US Patent 6,027,883 to Herrnstadt and incorporated herein by reference. The contamination of samples by nuclear DNA can be eliminated by purifying the isolated mt-DNA on a CsCI gradient prior to PCR amplification. This is particularly preferred where the nuclear DNA is believed to contain pseudogenes (nucleic acid segments incorporated within nuclear DNA that appear to be the same as or very similar to sequences of mt-DNA). This will help ensure that nuclear DNA is not inadvertently amplified or otherwise acted upon in a way that would interfere with reactions involving the target mt-DNA. It is also worth noting that any procedure for decreasing nuclear DNA contamination of mt-DNA samples intended for PCR amplification can be compromised if the primers selected favor the nuclear sequence. Thus, the judicious use of primers is also important in this regard and is discussed more fully below.

Commercially available kits for extracting and purifying mt-DNA are available and can also be used to good effect in the practice of this invention. For example, the alkaline lysis miniprep procedure is a simpler technique for the purification of mt-DNA. This technique is used through the application of the "WIZARD MINIPREPS DNA PURIFICATION SYSTEM" (commercially available from Promega Corp.). Numerous other mt-DNA extraction kits are readily available from companies such as Qiagen Corporation of Germany (preferred for blood samples) and Waco Corportion of Japan (preferred for tissue).

## Amplification and Reagents

The methods of this invention all employ PCR. The preferred methods of this invention employ short PCR protocols ("short PCR"). So-called "Long PCR" is not used. This ensures that wild type sequences of the size and type described herein are not amplified and hence falsely reported.

Hybridizing conditions should enable the binding of primers to the single nucleic acid target strand. As is known in the art, the primers are selected so that their relative positions along a duplex sequence are such that an extension product synthesized from one primer, when the extension product is separated from its template (complement) serves as a template for the extension of the other primer to yield a replicate chain of defined length. With this in mind, the reagents employed in the methods and kits of this invention include the following components.

1. Cleavage Reagents. Preferred cleavage reagents are restriction enzymes. Most preferred are restriction endonucleases. As noted above, other substances that can shear or cleave nucleic acids within a deletion sequence can also be used.
2. Amplification Reagents.

    a. PCR Reagents. Typical short PCR reagents are used. These include $Mg^{2+}$ containing solution (preferably $MgCl_2$), dNTP (each type), one polymerase having minimal 3' proofreading capability, and a signaling reagent such as the molecular beacons described in US Patent 6,150,097 (incorporated herein by reference). Preferably, the polymerase is a thermostable polymerase such as Taq polymerase. However, where the amplification technique is isothermal, the polymerase need not be thermostable. Primers specific to the sequences to be amplified using short PCR and buffers such as those containing Tris-hydroxyethylaminomethane ("TRIS") and KCl are also employed. Quantities and conditions for storage and application are all standard for short PCR amplification.

    Notably, typical long PCR reagents are not employed in the methods of this invention. That is, a proofreading polymerase is not employed, and the polymerase used is much less processive than a polymerase used in long PCR. Typically, it is present at an activity of about [1u] whereas that of long PCR is [2-5u]. Other reagents such as DMSO and glycerol used primarily to stabilize the polymerases used in long PCR are undesirable in the short PCR methods practiced in this invention. Further, TRIS buffer is preferred in the short PCR practiced with the inventive methods herein whereas TRICINE buffer would otherwise be used with long PCR techniques.

    As noted above, it is important that the primers be properly selected. This is a pragmatic issue that depends on the deletion sequence that is being targeted. It is desirable to have wild type DNA attacked by cleavage reagent (rendering it un-amplifiable when flanked by mutant primers). Thus, primer sequences that are to hybridize to the upstream portion of the sample nucleic acid are chosen to be sufficiently distant from those that are to hybridize to the downstream portion so that an uncleaved nucleic acid that is not the target of the assay does not amplify. This depends on the size and nature of the deletion sequence. Generally, the longer the deletion sequence, the less is the concern over this issue. One of ordinary skill will recognize how to manipulate primers in light of this concern and can readily craft them using only routine experimentation. The preferred primers of this invention are set forth in the sequence listings below.

b. The signal reagent referred to above is used to indicate that the target (and, optionally, controls) have been amplified. Numerous such signaling reagents are available including molecular beacons, "TAQMAN" probes (commercially available from PE Biosystems), Peptide Nucleic Acid ("PNA"), and DNAzyme probes. Alternatively the probe can be linked to one member of a pair of binding partners, e.g. biotin, and the label can be an avidin or strepavidin linked enzyme, such as a peroxidase, alkaline phosphatase or glucose oxidase. The label of different probes used in the same assay may be the same or different. Real time ("real-time" monitoring) so as to permit quantitation based on cycle of first detection of detectable fluorescent signal above a background is preferably done using molecular beacons or TaqMan probes or some equivalent homogeneous probe. Enzyme-labeled probes are used heterogeneously following PCR.

Where molecular beacons are used as probes in this invention, the references to typical probe lengths, stem lengths, fluorophores, and quenchers (e.g. DABCYL) described in US 6,150,097 can be applied. DABCYL is the preferred quencher and FAM (6-carboxyfluorescein) is the preferred fluorophore in single color molecular beacon systems.

As one skilled in the art will appreciate, other detection schemes are also available. These include, for example, detection techniques that are dependent on the size or molecular weight of the amplicon or nucleic acid fragment. Gel electrophoresis and capillary electrophoresis are examples of such techniques.

3. Amplification Conditions. Typical short PCR amplification conditions are used. These are the standard conditions one skilled in the art would apply when conducting PCR other than long PCR. Nonetheless, it is worth noting that annealing-extension times are much reduced relative to long PCR conditions. Typically, they are less than a minute and preferably they are about 30 seconds. This reduces the testing time required for commercial application of this invention.

The kits of this invention include packaged amplification reagents, signal reagents and cleavage reagents.

**Methods of the Invention**

Practicing the methods of the invention requires the isolation of a nucleic acid from a sample of interest, preferably mt-DNA. Each strand of the DNA molecule can be characterized as having an upstream zone, a downstream zone and a sequence between the upstream zone and the downstream zone (which sequence is deleted in mutant DNA). In wild type DNA, the deletion sequence can be cleaved in the presence of a cleavage reagent. Mutant DNA can be characterized as having an upstream zone and a downstream zone but, as noted above, lacks the deletion sequence found in wild type DNA. Thus, the presence of the cleavage reagent directed to such a deletion sequence found only in the wild type DNA has no cleaving effect on mutant DNA.

In one embodiment of this invention (preferably, where the target is mutant DNA with a 5kb deletion), the nucleic acid sample that has been isolated is divided into a first aliquot and a second aliquot. The first aliquot is contacted with a cleavage reagent, thereby forming a mixture. The mixture contains cleaved wild type DNA that cannot be amplified if mutant primers flanking the cut are used. However, if mutant DNA having the deletion is present, uncleaved mutant DNA can be amplified. This mixture is contacted with a forward primer specific for a portion of the upstream zone common to both mutant DNA and wild type DNA. To this mixture is added a reverse primer complementary to the downstream zone of the mutant DNA and each of four different nucleoside triphosphates as well as a DNA polymerase, under conditions such that only the mutant DNA is amplified.

The second aliquot is also contacted with a forward primer specific for a portion of the upstream zone of both DNA molecules and a reverse primer specific for a portion of the deletion sequence of the wild type DNA molecule. It is also contacted with four different nucleoside triphosphates and a DNA polymerase. This is conducted under conditions such that the DNA is amplified. Since no cleavage reagent is added, and since the sequence separating the priming site is short, wild type DNA that can hybridize to the primers will be amplified irrespective of the presence of deletion sequences. But mutant DNA molecules lack a reverse priming site complementary to this reverse primer, its site being located within the deletion sequence. The mutant DNA thus remains un-amplified. Therefore, only the wild type DNA is amplified. Accordingly, the second aliquot can serve as a positive control for the wild type DNA.

The aliquots are amplified in the presence of an appropriate signal reagent directed to detecting amplified species. More than one signal reagent may be used to indicate the presence of both the wild type DNA in the control and the mutant DNA in the first aliquot. This is readily done, for example, using molecular beacons with different fluorophors bound to beacons that are complementary to either the deletion sequence or, for example, the sequence at the interface of the mutant DNA segments that are spliced together where the deletion sequence would otherwise be.

A sample may be prepared that is suspected of comprising:

a) a sense wild type DNA strand containing an upstream zone, a downstream zone and a deletion sequence between the upstream zone and the downstream zone, the deletion sequence comprising an upstream zone and a downstream zone relative to a cleavage site,

b) an antisense wild type DNA strand complementary to the sense DNA strand comprising an upstream zone complementary to the downstream zone of the sense DNA molecule, a downstream zone complementary to the upstream zone of the sense DNA molecule, and a deletion sequence complementary to the deletion sequence of the sense DNA molecule, the deletion sequence of the antisense DNA molecule comprising an upstream zone complementary to the downstream zone of the deletion sequence of the sense DNA molecule and a downstream zone complementary to the upstream zone of the deletion sequence of the sense DNA molecule,

c) a sense mutant DNA strand comprising the upstream zone of the sense DNA strand and the downstream zone of the sense DNA strand, wherein the deletion sequence is not present (that is, the mutant DNA has a deletion mutation),

d) an antisense mutant DNA strand complementary to the sense mutant DNA strand, comprising an upstream zone complementary to the downstream zone of the sense mutant DNA strand, and a downstream zone complementary to the upstream zone of the sense mutant DNA strand.

The sample is divided into two aliquots. One aliquot is contacted with a cleavage reagent, specific for the cleavage site(s) of the deletion sequence, thereby forming a mixture of cleaved and uncleaved DNA molecules. The cleaved DNA molecules should not amplify in subsequent steps when mutant primers are used. The mixture of cleaved and uncleaved DNA molecules is contacted with a forward primer specific for a portion of the upstream zone of both the wild type DNA and the mutant DNA molecules; and a reverse primer specific for the downstream zone of the mutant DNA molecule. To this mixture is added four different nucleoside triphosphates, and a DNA polymerase, under short PCR conditions such that only the mutant DNA is amplified. Most, if not all, of the wild type DNA molecules will have been cleaved by the cleavage reagents thereby rendering them incapable of amplification when the primers for the mutant sequence are used (in the event that any wild type DNA escaped cleavage, they would also be incapable of amplification due to the inability to bridge the distance between priming sites using short PCR techniques according to the process of this invention). A probe specific for a sequence within the amplified portion of the mutant DNA molecules is added. This probe is labeled or capable of being labeled.

The second aliquot (in the absence of a cleavage reagent) is contacted with a forward primer specific for a portion of the upstream zone of both the wild type DNA molecule and the upstream zone of mutant DNA molecule and a reverse primer directed to a site within the deletion sequence of the wild type DNA, four different nucleoside triphosphates and a DNA polymerase, under conditions such that the wild type DNA is amplified. A probe specific for a sequence of the deletion sequence of the sense or antisense wild type DNA molecules is added. This probe is labeled or is capable of being labeled, preferably in a different manner than that of the probe in the case of the first aliquot.

The labels of each probe are then detected as cycling proceeds as a measure of the presence or amount of each type of DNA.

The deletion sequence should be sufficiently long (preferably, greater than 1kb) that it is not readily amplifiable using primers specific for the termini under the short PCR conditions employed in the process of this invention. The reverse primer for the mutant sequence is chosen to be sufficiently distant from the forward primer that even if some wt material survives restriction the wt sequence will not be amplified.

The drawings further illustrate the practice of this invention. In Fig. 1 (top), an amplicon is generated by a forward primer that binds to a priming site located in the upstream portion of the wtDNA molecule. The wildtype reverse priming site is located in the deletion sequence. The wildtype probe targets a site located within the deletion sequence. The bottom drawing of the figure shows the aliquot directed to muDNA. An amplicon is generated by a forward primer located in the upstream portion of the DNA molecule. The mutant priming site is located in the downstream portion while the mutant probe targets a region within the upstream portion. Fig. 1 illustrates methods particularly preferred for the detection of 5kb deletion sequences.

In Fig. 2 (top), an amplicon is generated by a forward primer that binds to a priming site located in the deletion sequence of the wtDNA molecule. The wildtype reverse priming site is located in the downstream portion of the DNA molecule. The wildtype probe targets a site located within the deletion sequence. The bottom drawing of the figure shows the aliquot directed to muDNA. An amplicon is generated by a forward primer located in the upstream portion of the DNA molecule. The mutant reverse priming site is located in the downstream portion while the mutant probe targets a region within the downstream portion. Fig. 2 illustrates methods particularly preferred for the detection of 7kb deletion sequences. In both figures, a and b refer to splice points. These are points at which a deletion, if present, would begin and end. Thus, for example, mtDNA with a 5kb deletion could have 5kb missing, starting at a and ending at b, that would not be missing in the case of wtDNA.

As a check on the presence of amplifiable DNA and as a control to ensure that amplification can occur, primers and probes appropriate for a conserved DNA sequence can be added to the contents of either or both the wild type and mutant aliquots. Thus amplification can involve co-amplification of this conserved region with either the wild type sequence (in the case of the wild type aliquot), or the mutant sequence (in the case of the mutant aliquot).

In the most preferred embodiments of this invention, reaction conditions are further controlled to minimize mis-priming that can result in the production and amplification of side products. These conditions are described by way of example in Example 7 and Example 8. In one of the methods, illustrated in Example 8, the thermal profile can be modified to include very stringent conditions initially. They are followed by less stringent cycles during which beacons are monitored in real-time. Indeed, in Example 8 the thermal profile was modified to include 10 initial cycles of PCR conducted under very stringent conditions (i.e., the anneal-extend temperature was selected to be 72°C). These early stringent PCR cycles were followed by 20 cycles of PCR conducted under less stringent conditions (an anneal step of 51°C was used, during which fluorescence from the probe was monitored in real-time as cycling proceeded). In a second approach, a second beacon directed at an amplicon located near the opposite end of each deletion sequence can be introduced. This confirms the presence of the desired product, and not just some fragement thereof. This is illustrated in Example 7 in which molecular beacons were directed at the downstream region of the 5 kb deletion sequence and the upstream region of the 7 kb deletion sequence, respectively.

**Examples:**

The following conditions and reagents were used throughout the examples (unless otherwise indicated).

| | |
|---|---|
| PCR MasterMix | |
| [Mg++] | (as $MgCl_2$), 3mM (except for Example 1, which was 4.5 mM); from Perkin-Elmer |
| [dNTP] | 0.2 mM each, from Boehringer-Mannheim |
| [Taq pol] | 1u/reaction well of ~ 50 ul |
| [beacon] [each primer] | 0.2 uM (except for Examples 2 & 3, where it was 0.083 uM) 0.15 uM |
| [buffer] | 10 mM Tris-HCl, pH 8.3, 50 mM KCl; no internal ROX standard dye; (obtained commercially from Perkin-Elmer). |

Restriction enzymes ScaI and PleI were purchased from New England BioLabs. Specimens were initially digested in restriction-specific buffers supplied by the vendor using 1 u of enzyme/ug of DNA as measured spectrophotometrically (except for PleI, where 15 u/1 ug of DNA was used). Reaction mixtures were incubated at 37 degrees C for 1 hour, then heat inactivated at 80 degrees C for 20 minutes. In all cases, 2 ul of digest were used per PCR well.

DdeI was purchased from Gibco. Two ul of enzyme were used to restrict 4 ul of mt-DNA at a concentration of 0.24 u/ug, diluted into 4 ul of 10X buffer diluted with 30 ul of deionized water. Incubation was at 37 degrees C for 3.5 hours.

Primers and probes were synthesized by Research Genetics and TriLink according to sequence specifications specified by the inventors.

PCR thermal profile:

89 degrees/ 15 sec.
55 degrees/ 15 sec.
58 degrees/ 10 sec.
62 degrees/ 15 sec

Instrument:

Perkin-Elmer ABI 7700 Sequence Detector

mt-DNA extraction kits were purchased from Qiagen Corporation and mt-DNA was extracted as directed from the whole blood of volunteers, except for mt-DNA standards (described below), which were purchased from the National Institute of Standards and Technology, Washington, D.C.

**Example 1: Amplification and Detection of the Control Sequence**

A control sequence was prepared as a reference both to test for the presence of mt-DNA and a standard relative to which the 5 kb and 7 kb sequences were measured..

The oligonucleotide target had the following sequence (bases 271- 377):

5'- cacagacatc ataacaaaaa atttccacca aacccccccct cccccgcttc tggccacagc acttaaacac atctctgcca aaccccaaaa acaaagaacc ctaacac-3' Seq. ID. No 1.

The forward primer used in this example was 24 nt in base length and had the sequence (bases 271- 294): 5'-cac aga cat cat aac aaa aaa ttt-3' Seq. ID. No 2.

The reverse primer used in this example was 25 nt in base length (bases 353 - 377) and had the sequence: 5'-gtg tta ggg ttc ttt gtt ttt gggg-3' Seq. ID. No 3.

The probe having the sequence listed below was a molecular beacon, having a 6nt long stem sequence (underlined). It probes bases 321 - 341.

5'FAM -gcg agc tct ggc cac agc act taa acc c gct cgc dabcyl-3' Seq. ID. No 4.

The restriction enzyme used for this purpose was BamHI, which recognizes the following sequence:
5' ggacc ... 3'
3' cctagg 5' ,

It is one of many restriction enzymes that cleaved mt DNA outside of the control region.

The control sequence was amplified in the presence of the materials described above and a plot was made of the logarithm of the concentration of the control sequence, versus PCR cycle number at which fluorescence first became detectable over background ("$C_t$"). This was done for a dilution series of different copy levels ranging in number from 3 x $10^0$ copies to 3x$10^8$ copies of mt-DNA . A similar plot was made using amplification products from unrestricted sequences. With restriction it was possible to obtain a regression line spanning approximately 7 decades in copy number, but without restriction, a linear regression line was obtained that spanned only 3 decades. This example shows the ability to use primers and probes as described for the amplification and detection of mtDNA. This was shown despite the fact that mtDNA is circular, covalently bonded at its ends, and well hybridized. This example also shows that improved amplification can be attained through the use of cleavage reagents.

**Example 2: Amplification and Detection of the wt-DNA.**

The wild-type oligonucleotide target used this example has the following sequence (bases 8344 - 8670):

agaaccaaca cctctttaca gtgaaatgcc ccaactaaat actaccgtat ggcccaccat aattaccccc atactcctta cactattcct catcacccaa ctaaaaatat taaacacaaa ctaccaccta cctccctcac caaagcccat aaaaataaaa aattataaca aaccctgaga accaaaatga acgaaaatct gttcgcttca ttcattgccc ccacaatcct aggcctaccc gccgcagtac tgatcattct atttcccct ctattgatcc ccacctccaa atatctcatc aacaaccgac taatcaccac ccaacaatga Seq. ID. No 5.

The forward primer used in this example was 27 nt in base length (bases 8344 - 8369) and had the sequence: 5'-acc aac acc tct tta cag tga aat gcc-3' Seq. ID. No 6

The reverse primer used in this example was 21 nt in base length (bases 8650 - 8670) and had the sequence: 5'-tca ttg ttg ggt ggt gat tag-3' Seq. ID. No 7

The wild-type probe had the sequence listed below. It was a molecular beacon (bases 8490 - 8510), having a 6nt long stem sequence (underlined):
5'FAM -ccg tcg cct ccc tca cca aag ccc ata aa cga cgg dabcyl-3' Seq. ID. No 8.

The restriction enzyme used to cleave this deletion sequence was ScaI, which recognizes the following sequence:

5' agtat...... 3'
3'tcatga......5'

Seal does not cut the control region, which can be co-amplified with other sequences if desired.

A PCR reaction using the materials described above was conducted. One set of samples was not contacted with cleavage reagents while another was so contacted. A plot of fluorescence versus cycle number for the 5 kb wt sequence in the presence and absence of target DNA indicated that the sample unrestricted with ScaI showed an increase in fluorescence beginning at cycle 16. This showed that the sample contained a sizable amount of 5kb wt mt-DNA. A similar plot using the sample restricted with ScaI resulted in a shift in fluorescence vs. cycle number ~6 cycles to the right, indicating that the number of wt molecules was reduced by restriction by a factor of - $2^6$ = 64 fold.

**Example 3: Amplification and Detection of DNA with a 5kb Deletion**

Master mix:

[Buffer] 10 mM Tris-HCl, pH 8.3, 50 mM KCl from Perkin-Elmer
[MgCl2] 5 mM from Sigma
[dNTP,each] 0.2 mM from Kodak
[Taq pol] 1u/reaction well (50 ul)
[5Mb3-TET (beacon)] 0.1 mM (if used)
[Primer, each] 0.17 uM

Cleavage Reagent:

The restriction enzyme Hind III was purchased from New England BioLabs. The suggested standard reaction conditions were used, except with approximately 5U of enzyme per ug of DNA, instead of 1U/ug.

In this example, the following PCR profile was used for a first round of amplification: 92 degrees/15 sec., 71 degrees/45 sec. for 40 cycles.

The above master mix, without the beacon present, was used for PCR with the following Primers:
5' accaaca cctctttaca gtgaaatgcc 3' Seq. ID No. 6
5' tgtatg atatgtttgc ggtttcgatg at 3' Seq. ID No. 9

100 ng of Hind III-digested DNA was also included.

The products from the above PCR were examined by agarose gel electrophoresis. There were no product gel bands observed in any lane.

For the next part of this experiment, 1 ul of target DNA was removed from each well in the above PCR reaction. This DNA was placed into a new well containing new master mix and the following primers:
5' gcc ccaactaaat actaccg 3' Seq. ID No. 10
5' gatgtggtctt tggagtagaaacctg 3' Seq. ID No. 11

The PCR profile used for this second amplification was: 92 degrees/15 sec., 69 degrees/45 sec for 40 cycles.

Agarose gel electrophoresis was performed on the samples obtained following two rounds of PCR as described above. Gel bands of size compatible with deletions of the 5 kb region (MW of approximately 174 bp) were seen in all samples examined. No primer-dimer bands were observed.

In the last part of this experiment, 1 ul of target DNA was removed from the PCR amplification that had been performed at an annealing-extension temperature of 71oC (the first PCR round). This sample was added to a reaction tube containing new master mix, the following primers were used:
5' gcc ccaactaaat actaccg 3' Seq. ID No. 10
5' gatgtggtctt tggagtagaaacctg 3' Seq. ID No. 11
along with the following beacon: 5' TET-ccgctcgaaa ggtattcctg ctaatgctag gctgccaatc gagcgg-Dabcyl 3' Seq. ID No. 12

This tube was then subjected to the following PCR conditions:

92 degrees/15 sec.
71 degrees/30 sec for 10 cycles, then
92 degrees/15 sec
57 degrees/ 10 sec
69 degrees//15 for 30 cycles.

A fluorescent signal from the probe was monitored at 57oC in real-time in the ABI PRISM 7700 analyzer.

Fluorescence versus cycle number was plotted for the 5 kb mu sequence, in the presence and absence of target DNA. The increase in fluorescence, which crossed the threshold after PCR cycle -10, indicated a positive beacon signal for the 5 kb deletion. This fluorescent signal is consistent with the observation of the corresponding gel band described earlier.

The products of the reamplified PCR were then visualized by agarose gel electrophoresis.

This figure demonstrated the presence of 5 kb mutant DNA in the samples, as seen by a major band of approximate MW 174 bp. Minor side products were also seen.

**Example 4: Amplification and Detection of wt-DNA.**

Wild-type oligonucleotide target had the following sequence (bases 16033 - 16213):

ggggaagc agatttgggt accacccaag tattgactca cccatcaaca accgctatgt atttcgtaca ttactgccag ccaccatgaa tattgtacgg taccataaat acttgaccac ctgtagtaca taaaaaccca atccacatca aaaccccctc cccatgctta caagcaagta cag Seq. ID. No 13

Forward wt primer used in this example was 22 nt in base length (bases 16033-16054) and had the sequence: 5'-ggg gaa gca gat ttg ggt acc a -3' Seq. ID. No 14

The reverse primer was 20 nt in base length (bases 16194- 16213) and had the sequence:
5'-ctg tac ttg ctt gta agc at -3' Seq. ID. No 15

The WT probe having the sequence listed below (bases 16065- 16088) is a molecular beacon, having a 6nt long stem sequence (underlined):
5'FAM- <u>gcgtcg</u> gac tca ccc atc aac aac cgc tat <u>cga cgc</u>-dabcyl 3' Seq. ID. No 16

The restriction enzyme used to cleave this 7 kb deletion sequence was PleI, which recognizes the following sequence:
5'... gagtc ...3'
3'...ctcag ... 5 '

PleI does not cut the conserved region, which can be co-amplified.

A PCR reaction using the materials described above was conducted. One set of samples was not contacted with cleavage reagents while another was so contacted. A plot of fluorescence versus cycle number for the 7 kb WT sequence in the presence and absence of target DNA was made. This sample unrestricted with PleI showed an increase in fluorescence beginning at cycle number 17 indicating the presence of a sizable amount of 7 kb wild type mt-DNA in this specimen. A plot of fluorescence versus cycle number for the restricted sample showed a shift of ~5 cycles to the right, indicating that the number of WT molecules was reduced by restriction by a factor of ~ $2^5$ = 32 fold.

**Example 5: Amplification and Detection of DNA having a 7kb Deletion (Prophetic).**

Example 4 is repeated except that one aliquot of sample contains mtDNA with a 7kb deletion.

The mutant (7kb deletion) target sequence is:

5'-gccgcagtac tgatcattct atttccccct ctattgatcc ccacctccaa atatctcatc aacaaccg gctatgt atttcgtaca ttactgccag ccaccatgaa tattgtacgg taccataaat acttgaccac ctgtagtaca taaaaaccca atccacatca aaaccccctc cccatgctta caagcaagta cag –3' Seq. ID. No 17

The mutant probe is a molecular beacon (bases 16103 - 16127) having the sequence: 5' -<u>gcg tcg</u> ctg cca gcc acc atg aat att gta <u>cga cgc</u> dabcyl-3' Seq. ID. No 18

The forward mutant primer used in this example is 33 nt in base length (bases 8581- 8613) and has the sequence: 5'-gcc gca gta ctg atc att cta ttt ccc cct cta -3' Seq. ID. No 19

The muDNA amplifies and is detected by interrogation of the probe.

**Example 6: Calibration Line Comparison for NIST Human mt-DNA Standards**

The mt-DNA reference standards obtained from NIST and referred to in the first example above were used as standards in duplicate calibration experiments performed separately. The control region was amplified and detected using Seq. ID No 4 (5'FAM - gcg agc tct ggc cac agc act taa acc c gct cgc dabcyl-3') and associated primers. PCR was performed as described in Example 1; values of Ct were determined and plotted versus the logarithm of target copy number. The linear regression parameters obtained are as shown in Table 1, below. The intercept of the calibration curve is the number of cycles required to detect a single copy of target, as shown below.

The calibration equation is:

$$Ct = Intercept + Slope \times \log [copy \#]$$

When the copy # = 1, the log [1] = 0; hence,
Ct for a single copy is given by the intercept; hence the intercept indicates the number of PCR cycles required for single copy sensitivity.

**Table 1:** Calibration Parameters for NIST mt-DNA

| | Set 1 | Set 2 |
|---|---|---|
| Slope | -2.45 | -2.59 |
| Intercept | 46.7 | 45.8 |

This example shows the reproducibility of the procedure (the two intercepts differ by < 1 cycle, while the slopes differ by only 5%), and also indicates that the assay possesses single copy sensitivity at a level of 46 or 47 PCR cycles. Thus, the assay is reproducible and sensitive. Moreover, the establishment of a reliable calibration curve enables the quantitation of mutant DNA relative to wildtype DNA or relative to the control sequence. This can be particularly important in distinguishing between disease state or condition that is related to aging as opposed to disease state or condition related to other causes.

**Example 7: Testing two additional probes directed at 5 kb and 7 kb mutant amplicons**

Two beacons were targeted to each of two mutant amplicons produced by 5 kb and 7 kb deletions, respectively. These probes were tested in PCR according to conditions listed below. Two new primers were synthesized and used to amplify a portion of the wild type mt-DNA genome containing a binding site complementary to the probes.

The sequences of these primers and probes were as follows. The probe for the 5 kb deletion was: 5' TET-ccg ctc ga aag gta ttc ctg cta atg cta ggc tgc caa tc gag cgg-Dabcyl Seq. ID No 12

The probe for the 7 kb deletion was 5' TET-ccgctcg gccgcagtac tgatcattct atttcccct cta cgagcgg-Dabcyl 3' probe Seq. ID No 20

(Underlining denotes the stem region of hairpin probe)

The reverse primer used in conjunction with the probe for the 5kb deletion had the sequence: tgt atg ata tgt ttg cgg ttt cga tga t Seq. ID No 9

The forward primer had the sequence:
tac tca aaa cca tac ctc tca ctt caa cct. Seq. ID No 21

This primer was synthesized to obtain amplification at a site to which the probe could bind.

The forward primer used in conjunction with the probe for the 7kb deletion had the sequence:
gaa cga aaa tct gtt cgc ttc att cat tgc. Seq. ID No 22

The reverse primer had the sequence:
ctg tac ttg ctt gta agc at. Seq. No 15

This primer was synthesized to obtain amplification at a site to which the probe could bind.

The thermal profile used on the ABI 7700 sequence detector was as follows:
50 C for 5 sec
95 C for 1 min.
10 cycles of two-temperature PCR, viz.,

92 C for 15 sec (denaturation)
72 C for 30 sec (annealing-extension);
followed by 30 cycles of three-temperature PCR; viz.
92 C for 15 sec (denaturation)
51 C for 10 sec (the annealing step during which beacons were read)
72 C for 15 sec. (the extension step).

The PCR mastermix consisted of :
10 mM TRIS, pH
5 mM MgCl2,
50 mM KCl;
0.2 mM, each dNTP,
~ 2 u of Taq DNA polymerase

Using a fluorescence threshold of 29 fluorescence units, target was first detected in real time at - 7 PCR cycles using the beacon for the 5kb deletion. Using a threshold of 39 fluorescent units, target was first detected at ~16 PCR cycles using the beacon for the 7kb deletion.

The products of these PCR reactions were electrophoresed on a standard agarose gel and stained with ethidium bromide. Bands were observed corresponding to amplicons of the correct size (~ 126 base pairs for the 5 kb and ~ 236 base pairs for the 7 kb, respectively). A no-template control lane corresponding to the blank reactions for the 7 kb deletion were completely free of bands, although a small amount of what appeared to be primer-dimer side product was observed in the lane corresponding to the blank for the 5 kb deletion.

**Example 8: Stringent Early PCR Cycles**

As in earlier examples, mt DNA was subjected to PCR real-time monitoring in the presence of mutant primer sets for both the 5 kb and 7 kb deletion sequences, except that two different thermal profiles were employed.

The first thermal profile was:
50 C for 5 sec, and 95 C for 5 sec,
followed by 40 cycles of:
89 C for 15 sec,
55 C for 15 sec,
58 C for 10 sec, and
62 C for 15 sec.

The primer/beacon sets were:
5 kb Mutant:
Beacon 5' FAM gcgtcg cat cac cca act aaa aat att aaa cac cgacgc -Dabcyl 3' Seq. ID No. 23
Forward Primer 5' accaaca cctctttaca gtgaaatgcc 3' Seq. ID No. 6
Reverse Primer 5' ctg cta atg cta ggc tgc caa t 3' Seq. ID No. 24 7 kb Mutant:
Forward Primer 5' gccgcagtac tgatcattct atttccccct cta 3' Seq. ID No. 19

Following PCR, amplified products were electrophoresed on agarose gels under standard conditions, and stained with ethidium bromide to reveal the size distribution of the reaction products. The gel exhibited numerous bands indicative of non-specific priming.

The second PCR thermal profile commenced with 10 cycles using a high [stringent] annealing temperature, followed by 30 cycles of real-time monitoring under less stringent conditions; i.e., at a lower annealing temperature. The primers/beacons used were:

<u>5 kb Mutant:</u>

Beacons
5' FAM-cgccgcct catcacccaa ctaaaaatat taaacacaaa ctaccacc ggcggcg-Dabcyl 3' Seq. ID No. 25
Forward Primer 5' gcc ccaactaaat actaccg 3' Seq. ID No. 10
Reverse Primer 5' gatgtggtctt tggagtagaaacctg 3' Seq. ID No. 11

<u>7 kb Mutant:</u>

Forward Primer 5' gccgcagtac tgatcattct atttccccct cta 3' Seq. ID No. 19
Beacon 5' FAM-cctgcgg ctgccag ccaccatgaa tattgtacgg ta ccgcagg-Dabcyl 3' Seq. ID No. 26

The thermal profile was:

50 C for 5 sec,
95 C for 60 sec;
10 cycles of:
95 C for 15 sec,
71 C for 30 sec;
followed by 30 cycles of:
92 C for 15 sec,
51 C for 10 sec (the annealing step during which fluorescence was read),
71 C for 15 sec.

Once again, amplified product was electrophoresed on a gel, stained with ethidium bromide, and the resulting bands compared to those of markers of known molecular weight.

In contrast with the results obtained under the previous thermal cycling conditions, no fluorescence above background was observed during real-time monitoring. Moreover, the gel results showed a complete absence of bands. Therefore, no mutant DNA was detected in this specimen.

**Example 9: Use of Hind III restriction (Prophetic)**

Although PleI and Sca1 can be used for restricting wt DNA as described in earlier examples, it is convenient to use just one restriction enzyme capable of cleaving both the 7 kb and 5 kb wt mt-DNA within their respective deletion sequences. Hind III, which cuts the following sequence:
AAGCTT
TTCGAA,
is used for this purpose. It does not cut the control region; hence, when it is desired to quantitate deletions relative to the control region, Hind III is especially preferred. It is incubated for 1 hour at 37 C with DNA at a level of ~1 u/ug DNA, as described by the manufacturer (New England Nuclear).

## Claims

**1.** A method of detecting mutant DNA having a deletion of no less than 4 kb comprising:

a) dividing a sample comprising nucleic acid suspected of containing a mixture of mutant DNA and wild type DNA into a first aliquot and a second aliquot;

b) contacting the first aliquot with a cleavage reagent specific for the deletion sequence, thereby forming a mixture;
c) contacting the mixture of step b) with:

a forward primer complementary to a priming site upstream of the deletion sequence;
a reverse primer complementary to a priming site downstream of the deletion sequence;
each of four different nucleoside triphosphates; and
a DNA polymerase,
under short PCR conditions, in which the sequences that are amplified are less than about 1 kb, such that only the mutant DNA is amplified;

d) contacting the second aliquot with:

a forward primer complementary to a priming site upstream of the deletion sequence;
a reverse primer complementary to a priming site within the deletion sequence;
each of the four different nucleoside triphosphates, and
a DNA polymerase
under conditions such that the wild type DNA is amplified; and

e) detecting the presence of the amplified mutant DNA.

**2.** A method of detecting mutant DNA having a deletion of no less than 4 kb comprising:

a) dividing a sample comprising nucleic acid suspected of containing a mixture of mutant DNA and wild type DNA into a first aliquot and a second aliquot;
b) contacting the first aliquot with a cleavage reagent specific for the deletion sequence, thereby forming a mixture;
c) contacting the mixture of step b) with:

a reverse primer complementary to a priming site downstream of the deletion sequence;
a forward primer complementary to a priming site upstream of the deletion sequence;
each of four different nucleoside triphosphates; and
a DNA polymerase, under conditions such that only the mutant DNA is amplified;

d) contacting the second aliquot with:

a forward primer complementary to a priming site within the deletion sequence;
a reverse primer complementary to a priming site downstream of the deletion sequence;
each of four different nucleoside triphosphates, and a DNA polymerase, under conditions such that the wild type DNA is amplified; and

e) detecting the presence of the amplified mutant DNA.

**3.** A method of quantitating mutant nucleic acids having deletion sequences of no less than 4 kb comprising:

a) dividing a sample comprising nucleic acid suspected of containing a mixture of mutant DNA and wild type DNA into a first aliquot and a second aliquot;
b) contacting the first aliquot with a cleavage reagent which is specific for the deletion sequence, thereby forming a mixture;
c) contacting the mixture of step b) with:

a forward primer complementary to a priming site upstream of the deletion sequence;
a reverse primer complementary to a priming region downstream of the deletion sequence;
under short PCR conditions in which the sequences which are amplified are less than about 1 kb,

wherein the upstream priming site is sufficiently distant from the downstream priming site so that uncleaved wild-type nucleic acid does not amplify under said short PCR conditions;
d) contacting the second aliquot with either:

a forward primer which is complementary to a priming site upstream of the deletion sequence and a reverse primer which is complementary to a priming site within the deletion sequence; or

a forward primer which is complementary to a priming site within the deletion sequence and a reverse primer which is complementary to a priming site downstream of the deletion sequence, under short PCR conditions in which the sequences which are amplified are less than about 1 kb,

wherein the upstream priming site and the downstream priming site are no greater than 1 kb apart for the wild type DNA;

e) identifying the presence of amplicons of step c);

f) identifying the presence of amplicons of step d); and

g) quantitating the presence of amplicons of step c) by comparing the quantity of the mutant nucleic acid with the quantity of the non-mutant nucleic acid, wherein the quantitation of the presence of amplicons of step c) is relative to the amount of wild type nucleic acid present in the sample.

**4.** The method of claim 3 wherein quantitation is conducted by comparison to a standard.

**5.** The method of claim 3 or claim 4 wherein quantitation is conducted by real-time monitoring.

**6.** A kit for detecting or quantitating the occurrence of nucleic acid deletion sequences, wherein the kit comprises:

mutant PCR primers: wherein said mutant PCR primers are complementary to priming sites that are no less than 1 kb apart for the wild type DNA;

a cleavage reagent specific for the deletion sequence; and

wild type PCR primers which are complementary to priming sites that are no greater than 1 kb apart for the wild type DNA, wherein either:

the forward primer is complementary to a priming site upstream of the deletion sequence and the reverse primer is complementary to a priming site within the deletion sequence; or

the forward primer is complementary to a priming site within the deletion sequence and the reverse primer is complementary to a priming site downstream of the deletion sequence.

**7.** The kit of claim 6, further comprising one polymerase having minimal 3' proofreading capability at an activity of [1u].

**8.** The kit of claim 6 or claim 7, further comprising TRIS buffer.

**Patentansprüche**

**1.** Verfahren zum Nachweisen von Mutanten-DNA, die eine Deletion nicht kleiner als 4 kb aufweist, umfassend:

a) Teilen einer Probe, die eine Nukleinsäure umfasst von der vermutet wird, daß sie eine Mischung aus Mutanten-DNA und Wildtyp-DNA enthält, in ein erstes Aliquot und ein zweites Aliquot,

b) Kontaktieren des ersten Aliquots mit einem Spaltungsreagenz, das für die Deletionssequenz spezifisch ist, und dabei Bilden einer Mischung;

c) Kontaktieren der Mischung aus Schritt b) mit:

einem Vorwärts-Primer, der zu einer Primerbindestelle stromaufwärts der Deletionssequenz komplementär ist;

einem Rückwärts-Primer, der zu einer Primerbindestelle stromabwärts der Deletionssequenz komplementär ist;

jedem von vier verschiedenen Nukleosidtriphosphaten; und

einer DNA-Polymerase,

unter kurzen PCR-Bedingungen, in denen die Sequenzen, die amplifiziert werden, kleiner als etwa 1 kb sind, so daß nur die Mutanten-DNA amplifiziert wird;

d) Kontaktieren des zweiten Aliquots mit:

einem Vorwärts-Primer, der zu einer Primerbindestelle stromaufwärts der Deletionssequenz komplementär ist;
einem Rückwärts-Primer, der zu einer Primerbindestelle innerhalb Deletionssequenz komplementär ist;
jedem von vier verschiedenen Nukleosidtriphosphaten; und
einer DNA-Polymerase,
unter Bedingungen, so daß die Wildtyp-DNA amplifiziert wird; und

e) Nachweisen der Anwesenheit der amplifizierten Mutanten-DNA.

**2.** Verfahren zum Nachweisen von Mutanten-DNA, die eine Deletion nicht kleiner als 4 kb aufweist, umfassend:

a) Teilen einer Probe, die eine Nukleinsäure umfasst von der vermutet wird, daß sie eine Mischung aus Mutanten-DNA und Wildtyp-DNA enthält, in ein erstes Aliquot und ein zweites Aliquot,
b) Kontaktieren des ersten Aliquots mit einem Spaltungsreagenz, das für die Deletionssequenz spezifisch ist, und dabei Bilden einer Mischung;
c) Kontaktieren der Mischung aus Schritt b) mit:

einem Rückwärts-Primer, der zu einer Primerbindestelle stromabwärts der Deletionssequenz komplementär ist;
einem Vorwärts-Primer, der zu einer Primerbindestelle stromaufwärts der Deletionssequenz komplementär ist;
jedem von vier verschiedenen Nukleosidtriphosphaten; und
einer DNA-Polymerase,
unter Bedingungen, so daß nur die Mutanten-DNA amplifiziert wird;

f) Kontaktieren des zweiten Aliquots mit:

einem Vorwärts-Primer, der zu einer Primerbindestelle innerhalb der Deletionssequenz komplementär ist;
einem Rückwärts-Primer, der zu einer Primerbindestelle stromabwärts der Deletionssequenz komplementär ist;
jedem von vier verschiedenen Nukleosidtriphosphaten, und einer DNA-Polymerase, unter Bedingungen, so daß die Wildtyp-DNA amplifiziert wird; und

g) Nachweisen der Anwesenheit der amplifizierten Mutanten-DNA.

**3.** Verfahren zum Quantifizieren von mutanten Nukleinsäuren, die Deletionssequenzen nicht kleiner als 4 kb aufweisen, umfassend:

a) Teilen einer Probe, die eine Nukleinsäure umfasst von der vermutet wird, daß sie eine Mischung aus Mutanten-DNA und Wildtyp-DNA enthält, in ein erstes Aliquot und ein zweites Aliquot,
b) Kontaktieren des ersten Aliquots mit einem Spaltungsreagenz, das für die Deletionssequenz spezifisch ist, und dabei Bilden einer Mischung;
c) Kontaktieren der Mischung aus Schritt b) mit:

einem Vorwärts-Primer, der zu einer Primerbindestelle stromaufwärts der Deletionssequenz komplementär ist;
einem Rückwärts-Primer, der zu einer Primerbindestelle stromabwärts der Deletionssequenz komplementär ist;
unter kurzen PCR-Bedingungen, in denen die Sequenzen, die amplifiziert werden, kleiner als etwa 1 kb sind,

wobei die stromaufwärts gelegene Primerbindestelle ausreichend entfernt von der stromabwärts gelegenen Primerbindestelle ist, so daß nicht-gespaltene Wildtyp-Nukleinsäure nicht unter den kurzen PCR-Bedingungen amplifiziert wird;
d) Kontaktieren des zweiten Aliquots mit entweder:

einem Vorwärts-Primer, der zu einer Primerbindestelle stromaufwärts der Deletionssequenz komplementär ist und einem Rückwärts-Primer, der zu einer Primerbindestelle innerhalb der Deletionssequenz komplementär ist; oder

einem Vorwärts-Primer, der zu einer Primerbindestelle innerhalb der Deletionssequenz komplementär ist und einem Rückwärts-Primer, der zu einer Primerbindestelle stromabwärts der Deletionssequenz komplementär ist, unter kurzen PCR-Bedingungen, in denen die Sequenzen, die amplifiziert werden, kleiner als etwa 1 kb sind,

wobei die stromaufwärts gelegene Primerbindestelle und die stromabwärts gelegene Primerbindestelle nicht mehr als 1 kb für die Wildtyp-DNA auseinander sind;
e) Identifizieren der Anwesenheit der Amplicons aus Schritt c);
f) Identifizieren der Anwesenheit der Amplicons aus Schritt d); und
g) Quantifizieren der Anwesenheit der Amplicons aus Schritt c) durch Vergleichen der Quantität der mutanten Nukleinsäure mit der Quantität der nicht-mutanten Nukleinsäure, wobei die Quantifizierung der Anwesenheit der Amplicons aus Schritt c) relativ zu der Menge an der in der Probe vorhandenen Wildtyp-Nukleinsäure ist.

**4.** Verfahren nach Anspruch 3, wobei Quantifizierung durch Vergleich zu einem Standard durchgeführt wird.

**5.** Verfahren nach Anspruch 3 oder Anspruch 4, wobei Quantifizierung durch Echtzeit-Monitoring durchgeführt wird.

**6.** Kit zum Nachweisen oder Quantifizieren des Vorkommens von Nukleinsäure-Deletionssequenzen, wobei der Kit umfasst:

mutanten PCR-Primer: wobei die mutanten PCR-Primer den Primerbindestellen komplementär sind, die für die Wildtyp-DNA nicht weniger als 1 kb auseinander sind;
ein Spaltungsreagenz, das für die Deletionssequenz spezifisch ist; und
Wildtyp-PCR-Primer, die den Primerbindestellen komplementär sind, die für die Wildtyp-DNA nicht mehr als 1 kb auseinander sind, wobei entweder:

der Vorwärts-Primer zu einer Primerbindestelle stromaufwärts der Deletionssequenz komplementär ist und der Rückwärts-Primer zu einer Primerbindestelle innerhalb der Deletionssequenz komplementär ist; oder
der Vorwärts-Primer zu einer Primerbindestelle innerhalb der Deletionssequenz komplementär ist und der Rückwärts-Primer zu einer Primerbindestelle stromabwärts der Deletionssequenz komplementär ist.

**7.** Kit nach Anspruch 6, weiter umfassend eine Polymerase, die eine minimale 3' Korrekturlese-Fähigkeit bei einer Aktivität von [1 u] aufweist.

**8.** Kit nach Anspruch 6 oder Anspruch 7, weiter umfassend TRIS-Puffer.

## Revendications

**1.** Procédé pour détecter un ADN mutant ayant une délétion non inférieure à 4 kb, comprenant :

a) diviser un échantillon comprenant un acide nucléique suspecté de contenir un mélange d'ADN mutant et d'ADN de type sauvage en une première aliquote et une deuxième aliquote ;
b) mettre la première aliquote en contact avec un réactif de coupure spécifique de la séquence de délétion, en formant ainsi un mélange ;
c) mettre le mélange de l'étape b) en contact avec :

une amorce sens complémentaire d'un site d'amorçage en amont de la séquence de délétion ;
une amorce antisens complémentaire d'un site d'amorçage en aval de la séquence de délétion ;
chacun de quatre nucléosides triphosphates différents ; et
une ADN polymérase,
dans des conditions de PCR courte, dans lesquelles les séquences qui sont amplifiées font moins d'environ 1 kb, de façon que seul l'ADN mutant soit amplifié ;

d) mettre la deuxième aliquote en contact avec

une amorce sens complémentaire d'un site d'amorçage en amont de la séquence de délétion ;
une amorce antisens complémentaire d'un site d'amorçage à l'intérieur de la séquence de délétion ;

chacun de quatre nucléosides triphosphates différents ; et
une ADN polymérase,
dans des conditions telles que l'ADN de type sauvage soit amplifié ; et

e) détecter la présence de l'ADN mutant amplifié.

**2.** Procédé pour détecter un ADN mutant ayant une délétion non inférieure à 4 kb, comprenant :

a) diviser un échantillon comprenant un acide nucléique suspecté de contenir un mélange d'ADN mutant et d'ADN de type sauvage en une première aliquote et une deuxième aliquote ;
b) mettre la première aliquote en contact avec un réactif de coupure spécifique de la séquence de délétion, en formant ainsi un mélange ;
c) mettre le mélange de l'étape b) en contact avec :

une amorce antisens complémentaire d'un site d'amorçage en aval de la séquence de délétion ;
une amorce sens complémentaire d'un site d'amorçage en amont de la séquence de délétion ; chacun de quatre nucléosides triphosphates différents ; et
une ADN polymérase,
dans des conditions telles que seul l'ADN mutant soit amplifié ;

d) mettre la deuxième aliquote en contact avec

une amorce sens complémentaire d'un site d'amorçage à l'intérieur de la séquence de délétion ;
une amorce antisens complémentaire d'un site d'amorçage en aval de la séquence de délétion ;
chacun de quatre nucléosides triphosphates différents ; et
une ADN polymérase,
dans des conditions telles que l'ADN de type sauvage soit amplifié ; et

e) détecter la présence de l'ADN mutant amplifié.

**3.** Procédé pour quantifier des acides nucléiques mutants ayant des séquences de délétion non inférieures à 4 kb, comprenant :

a) diviser un échantillon comprenant un acide nucléique suspecté de contenir un mélange d'ADN mutant et d'ADN de type sauvage en une première aliquote et une deuxième aliquote ;
b) mettre la première aliquote en contact avec un réactif de coupure qui est spécifique de la séquence de délétion, en formant ainsi un mélange ;
c) mettre le mélange de l'étape b) en contact avec :

une amorce sens complémentaire d'un site d'amorçage en amont de la séquence de délétion ;
une amorce antisens complémentaire d'une région d'amorçage en aval de la séquence de délétion ;
dans des conditions de PCR courte, dans lesquelles les séquences qui sont amplifiées font moins d'environ 1 kb,
le site d'amorçage en amont étant suffisamment distant du site d'amorçage en aval pour que l'acide nucléique de type sauvage non coupé ne s'amplifie pas dans lesdites conditions de PCR courte ;

d) mettre la deuxième aliquote en contact avec

soit une amorce sens qui est complémentaire d'un site d'amorçage en amont de la séquence de délétion et une amorce antisens qui est complémentaire d'un site d'amorçage à l'intérieur de la séquence de délétion ;
soit une amorce sens qui est complémentaire d'un site d'amorçage à l'intérieur de la séquence de délétion et une amorce antisens qui est complémentaire d'un site d'amorçage en aval de la séquence de délétion,
dans des conditions de PCR courte, dans lesquelles les séquences qui sont amplifiées font moins d'environ 1 kb,
le site d'amorçage en amont et le site d'amorçage en aval n'étant pas espacés de plus de 1 kb pour l'ADN de type sauvage ;

e) identifier la présence d'amplicons de l'étape c) ;

f) identifier la présence d'amplicons de l'étape d) ; et
g) quantifier la présence d'amplicons de l'étape c) en comparant la quantité de l'acide nucléique mutant avec la quantité de l'acide nucléique non mutant, la quantification de la présence d'amplicons de l'étape c) étant relative à la quantité d'acide nucléique de type sauvage présente dans l'échantillon.

**4.** Procédé selon la revendication 3, dans lequel la quantification est effectuée par comparaison avec un standard.

**5.** Procédé selon la revendication 3 ou la revendication 4, dans lequel la quantification est effectuée par surveillance en temps réel.

**6.** Trousse pour détecter ou quantifier l'apparition de séquences de délétion d'acides nucléiques, laquelle trousse comprend :

des amorces de PCR mutantes, lesquelles amorces de PCR mutantes sont complémentaires de sites d'amorçage qui ne sont pas espacés de moins de 1 kb pour l'ADN de type sauvage ;
un réactif de coupure spécifique de la séquence de délétion ; et
des amorces de PCR de type sauvage qui sont complémentaires de sites d'amorçage qui ne sont pas espacés de plus de 1 kb pour l'ADN de type sauvage, où :

soit l'amorce sens est complémentaire d'un site d'amorçage en amont de la séquence de délétion et l'amorce antisens est complémentaire d'un site d'amorçage à l'intérieur de la séquence de délétion ;
soit l'amorce sens est complémentaire d'un site d'amorçage à l'intérieur de la séquence de délétion et l'amorce antisens est complémentaire d'un site d'amorçage en aval de la séquence de délétion.

**7.** Trousse selon la revendication 6, comprenant en outre une polymérase ayant une capacité de correction en 3' minimale à une activité de [1u].

**8.** Trousse selon la revendication 6 ou la revendication 7, comprenant en outre un tampon TRIS.

Fig. 1

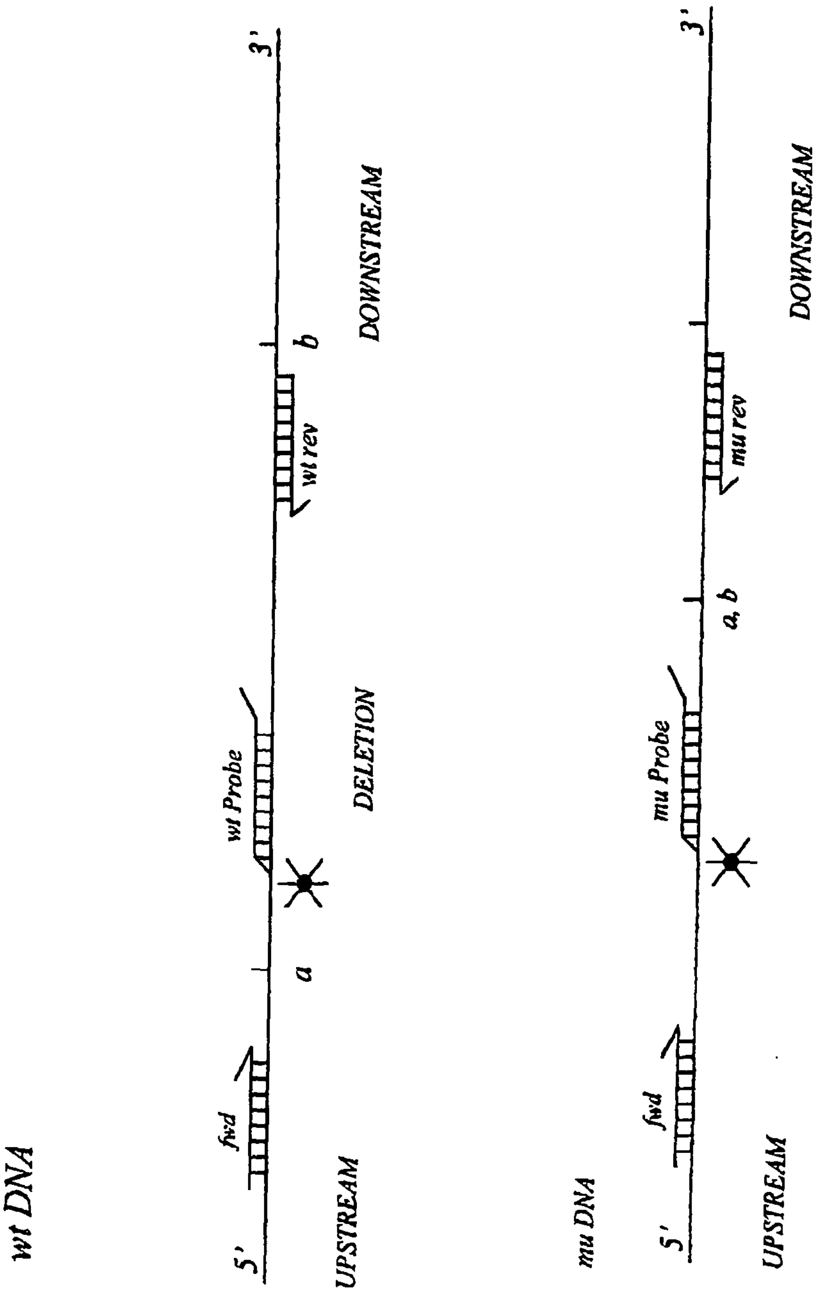
wt DNA
5'
fwd
UPSTREAM
a
wt Probe
DELETION
wt rev
b
DOWNSTREAM
3'
mu DNA
5'
fwd
UPSTREAM
mu Probe
a, b
mu rev
DOWNSTREAM
3'

Fig. 2

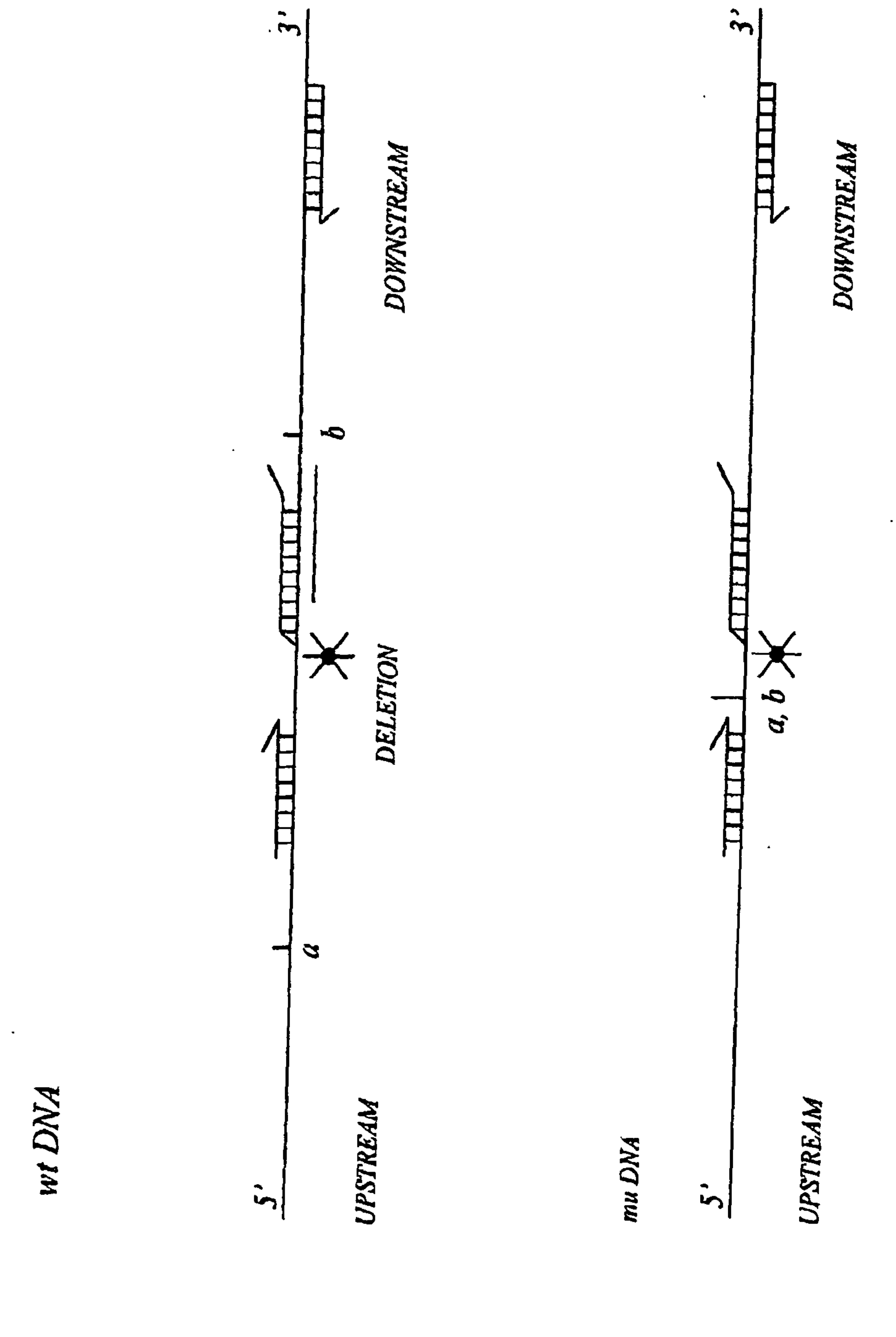
wt DNA
5'
3'
UPSTREAM
a
DELETION
b
DOWNSTREAM
mu DNA
5'
3'
UPSTREAM
a, b
DOWNSTREAM

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5436142 A, Wigler
- US 5919623 A, Taylor
- US 6110684 A, Kember
- US 5391480 A, Davis
- US 5958692 A, Cotton
- US 5876941 A, Landegren
- US 6001567 A, Brow
- US 6017701 A, Sorge
- WO 9632500 A, Todd
- US 6027883 A, Herrnstadt
- US 6150097 A
- EP 02254040 A
- US 09877748 B


### Non-patent literature cited in the description


- **MARIN-GARCIA, J. et al.** *Cardovascular Research,* 1996, vol. 31, 306-313
- **FUKUGAWA, N.M. et al.** *Free Radical Biology and Medicine,* 1999, vol. 27 (11/12), 1437-1443